(19) Europäisches Patentamt
European Patent Office
Office européen des brevets

(11) **EP 4 609 852 A1**

(12) **EUROPEAN PATENT APPLICATION**
published in accordance with Art. 153(4) EPC


(43) Date of publication:
**03.09.2025 Bulletin 2025/36**

(21) Application number: **23882637.4**

(22) Date of filing: **24.10.2023**

(51) International Patent Classification (IPC):
***A61K 8/86*** (2006.01) ***A23L 2/52*** (2006.01)
***A23L 33/10*** (2016.01) ***A23L 33/105*** (2016.01)
***A23L 33/12*** (2016.01) ***A61K 8/37*** (2006.01)
***A61K 8/39*** (2006.01) ***A61Q 19/00*** (2006.01)
***A61Q 19/10*** (2006.01) ***C07C 69/33*** (2006.01)
***C08G 65/332*** (2006.01) ***C08K 5/00*** (2006.01)
***C08L 71/00*** (2006.01)

(52) Cooperative Patent Classification (CPC):
**A23L 2/52; A23L 33/10; A23L 33/105; A23L 33/12; A61K 8/37; A61K 8/39; A61K 8/86; A61Q 19/00; A61Q 19/10; C07C 69/33; C08G 65/332; C08K 5/00; C08L 71/00**

(86) International application number:
**PCT/JP2023/038340**

(87) International publication number:
**WO 2024/090432 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.10.2022 JP 2022173544**
**31.01.2023 JP 2023013486**
**03.08.2023 JP 2023126814**



(71) Applicant: **Taiyo Kagaku Co., Ltd.**
**Mie 512-1111 (JP)**

(72) Inventors:
- **MATSUMOTO, Yoshiyuki**
  **Yokkaichi-shi, Mie, 512-1111 (JP)**
- **SAKANISHI, Yuichi**
  **Yokkaichi-shi, Mie, 512-1111 (JP)**
- **HIGUCHI, Tomonori**
  **Yokkaichi-shi, Mie, 512-1111 (JP)**

(74) Representative: **Vossius & Partner Patentanwälte Rechtsanwälte mbB**
**Siebertstrasse 3**
**81675 München (DE)**


(54) **POLYGLYCEROL FATTY ACID ESTER**


(57) A polyglycerol fatty acid ester which is an ester of a polyglycerol of which the ratio of a branched glycerol group represented by the following formula (1):

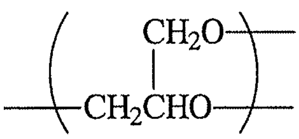

(1)

is from 14 to 60% and an average degree of polymerization is from 5 to 25, and a fatty acid having the number of carbon atoms of from 6 to 22, wherein an HLB is 13 or more. According to the present invention, the polyglycerol fatty acid ester having excellent capability of solubilizing poorly water-soluble substance can be provided, and the polyglycerol fatty acid ester can be suitably used in an application such as cosmetics and foodstuff.


EP 4 609 852 A1

## Description

### TECHNICAL FIELD

The present invention relates to a polyglycerol fatty acid ester, a solubilizing agent containing the compound, a composition containing the compound, a cosmetic and a foodstuff containing thereof.

### BACKGROUND ART

A poorly water-soluble substance such as perfume, essential oil, vegetable oil, hydrocarbon oil, ester oil and fat-soluble vitamin which is formulated to transparent cosmetics such as a skin toner or a beauty serum is solubilized with a surfactant. However, since perfume, essential oil, and vegetable oil have high polarity, a lot of surfactants need to be formulated to solubilize these. As a result, there has been a problem that usability worsens when applied to the skin as a cosmetic.

As a technique to solve such a problem, for example, Patent Publication 1 suggests a polyglycerol fatty acid ester in which a polyglycerol and a fatty acid are esterified, wherein the content of glycerol polymer having degree of polymerization of 3 or less is less than 10%.

In addition, also in foodstuff, transparency of appearance is required when solubilizing a poorly water-soluble substance to beverages. Further, since formulation of large amount of surfactant affects flavors, a reduction of the surfactant is needed.

### PRIOR ART REFERENCES

### PATENT PUBLICATIONS

Patent Publication 1: Japanese Patent Laid Open No. 2018-70529

### SUMMARY OF THE INVENTION

### PROBLEMS TO BE SOLVED BY THE INVENTION

However, a capability of solubilizing in Patent Publication 1 is not satisfactory, and a further improvement is required.

The purpose of the present invention is to provide a polyglycerol fatty acid ester having an excellent capability of solubilizing a poorly water-soluble substance.

### MEANS TO SOLVE THE PROBLEMS

The present invention relates to the following [1] to [6]:

[1] A polyglycerol fatty acid ester which is an ester of a polyglycerol of which the ratio of a branched glycerol group represented by the following formula (1):

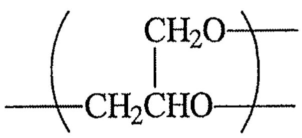

(1)

is from 14 to 60% and an average degree of polymerization is from 5 to 25, and a fatty acid having the number of carbon atoms of from 6 to 22, wherein an HLB is 13 or more.

[2] A solubilizing agent containing the polyglycerol fatty acid ester as defined in [1].

[3] A cosmetic composition containing the polyglycerol fatty acid ester as defined in [1] and a poorly water-soluble substance.

[4] Cosmetics containing the polyglycerol fatty acid ester as defined in [1] or the cosmetic composition as defined in [3].

[5] A composition for foodstuff containing the polyglycerol fatty acid ester as defined in [1] and a poorly water-soluble substance.

[6] Foodstuff containing the polyglycerol fatty acid ester as defined in [1] or the composition for foodstuff as defined in

[5].

EFFECTS OF THE INVENTION

According to the present invention, a polyglycerol fatty acid ester having an excellent capability of solubilizing a poorly water-soluble substance can be provided. Here, since solubilizing amount varies depending on the kind and the like of the poorly water-soluble substance, a polyglycerol fatty acid ester having improved capability of solubilizing as compared to a polyglycerol fatty acid ester formulated using commercially available polyglycerol (polyglycerol 7) described in the following Examples was judged as "excellent in capability of solubilizing."

BRIEF DESCRIPTION OF THE DRAWINGS

[FIG. 1] FIG. 1 is a 13C-NMR spectrum of Production Example 1.

MODES FOR CARRYING OUT THE INVENTION

As a result of intensively studying the above problems, the inventors found that a capability of solubilizing a poorly water-soluble substance became excellent by giving an appropriate branch structure to a polyglycerol which is a hydrophilic group of a polyglycerol fatty acid ester, thereby completing the present invention. As to such a mechanism, it is assumed that a micelle is likely to be formed by giving an appropriate branched structure to a polyglycerol which is a hydrophilic group.

The polyglycerol fatty acid ester of the present invention is an ester of a polyglycerol of which the ratio of a branched glycerol group represented by the above formula (1) is from 14 to 60% and an average degree of polymerization is from 5 to 25, and a fatty acid having the number of carbon atoms of from 6 to 22. The HLB of the polyglycerol fatty acid ester of the present invention is 13 or more, preferably from 13 to 19.5, more preferably from 13.5 to 19, even more preferably from 14 to 18.5, and even more preferably from 14.5 to 18, from the viewpoint of a capability of solubilizing the poorly water-soluble substance.

The HLB as used herein refers to a value which is measured by the following Griffin's calculation method. A saponification value and a neutralization value are measured by a known method.

$$HLB = 20\ (1 - S / A)$$

S: Saponification value of polyglycerol fatty acid ester
A: Neutralization value of raw material fatty acid

In the polyglycerol of the polyglycerol fatty acid ester of the present invention, the ratio of the branched glycerol group represented by the above formula (1) is from 14 to 60%, and preferably from 14 to 36%, and the average degree of polymerization is from 5 to 25, and preferably from 7 to 22, from the viewpoint of a capability of solubilizing the poorly water-soluble substance. In addition, the number of the branched glycerol group represented by the above formula (1) per polyglycerol molecule is preferably from 1 to 15 on average, and more preferably from 1 to 8 on average. Here, the ratio and the number of the branched glycerol group mentioned above can be adjusted by the raw material ratio at the time of manufacturing a polyglycerol. For example, in Examples described later, increasing the usage of the glycerol triglycidyl ether increases the ratio and the number of the branched glycerol group mentioned above, and decreasing the usage of the glycerol triglycidyl ether decreases the ratio and the number of the branched glycerol group mentioned above. Incidentally, the ratio and the number of the branched glycerol group mentioned above are measured by the method described in Examples described later.

The average degree of polymerization of the polyglycerol in the specification is an average degree of polymerization of the polyglycerol calculated from the hydroxyl value according to a terminal group analysis method, and is an average degree of polymerization calculated from (Formula 1) and (Formula 2).

Average degree of polymerization = (112.2 $\times$ 103 - 18 $\times$ hydroxyl value) / (74 $\times$ hydroxyl value - 56.1 $\times$ 103) (Formula 1)

Hydroxyl value = (a - b) $\times$ 28.05 / harvested amount of sample (g) (Formula 2)

a: Consumed amount of 0.5 N potassium hydroxide solution according to blank test (ml)
b: Consumed amount of 0.5 N potassium hydroxide solution according to main test (ml)

The hydroxyl value in the above (Formula 1) is calculated by (Formula 2) according to "Standard Methods for the Analysis of Fats, Oils and Related Materials (I), 1996, constituted by Japan Oil Chemists' Society" edited by a general incorporated association, Japan Oil Chemists' Society.

In addition, as to the polyglycerol according to the polyglycerol fatty acid ester of the present invention, total content of the glycerol and diglycerol is preferably from 0.01 to 30% by mass, and more preferably from 0.01 to 15% by mass, from the viewpoint of a capability of solubilizing the poorly water-soluble substance. The total content of the glycerol and diglycerol in the polyglycerol can be adjusted by control of the degree of purification by a column, a separate addition after purification or the like. In addition, the total content of the glycerol and diglycerol in the polyglycerol is measured by the method described in Examples mentioned later.

As to the fatty acid according to polyglycerol fatty acid ester of the present invention, the number of carbon atoms is from 6 to 22, preferably from 8 to 20, and more preferably from 8 to 18, from the viewpoint of a capability of solubilizing the poorly water-soluble substance. In addition, the number of carbon atoms may be from 6 to 12, from 8 to 12, from 14 to 22, from 14 to 20, from 14 to 18 and the like. Concretely, the fatty acid includes lauric acid, myristic acid, oleic acid, stearic acid, isostearic acid and the like, but not limited thereto.

The polyglycerol fatty acid ester of the present invention can be prepared by subjecting a polyglycerol with a fatty acid to an esterification reaction according to a general synthesis method. The polyglycerol fatty acid ester of the present invention can be suitably used as a solubilizing agent containing a polyglycerol fatty acid ester, a cosmetic composition and a composition for foodstuff containing a polyglycerol fatty acid ester and a poorly water-soluble substance, because the polyglycerol fatty acid ester has an excellent capability of solubilizing the poorly water-soluble substance. In addition, the polyglycerol fatty acid ester can be used in a known application as an application of a polyglycerol fatty acid ester. Hereinafter, a preferable embodiment of a solubilizing agent, a cosmetic composition, and a composition for foodstuff using the polyglycerol fatty acid ester of the present invention is explained.

(Solubilizing Agent)

The solubilizing agent of the embodiment contains the polyglycerol fatty acid ester of the present invention, and may optionally contain known components used in a solubilizing agent such as glycerol, 1,3-butylene glycol (BG), dipropylene glycol (DPG), and sugar alcohol.

The content of the polyglycerol fatty acid ester in the solubilizing agent of the embodiment is preferably from 20 to 80% by mass, and more preferably from 20 to 50% by mass.

(Cosmetic composition)

The cosmetic composition of the embodiment contains the polyglycerol fatty acid ester of the present invention and the poorly water-soluble substance.

The content of the polyglycerol fatty acid ester of the present invention in the cosmetic composition of the embodiment is preferably from 0.1 to 10% by mass, and more preferably from 0.1 to 5% by mass.

The poorly water-soluble substance as used herein means a substance which is hardly dissolved or dissolved in a slight amount into water, if any, and solubility to water is preferably 0.1% or less and more preferably 0.01% or less. For example, the poorly water-soluble substance includes perfume, essential oil, ester oil, hydrocarbon oil, animal and vegetable oil, fat-soluble vitamin, ceramide, preservative, antibacterial agent, composition containing these and the like.

The perfume includes hydrocarbon fragrance such as limonene, alcohol-based fragrance such as linalool, geraniol, and menthol (L-menthol), aldehyde-based fragrance such as citral, ketone-based fragrance such as β-ionone, phenolic fragrance such as eugenol, and the like.

The essential oil includes botanical essential oil such as lavender oil, eucalyptus oil, rose oil, jasmine oil, peppermint oil, anise oil, rosemary oil, and bergamot oil, and musk, civet musk, *kairyu,* ambergris and the like.

The ester oil includes isononyl isononanoate, isostearyl isostearate, cetyl 2-ethylhexanoate, 2-ethylhexyl palmitate, triethylhexanoin, glyceryl tri(caprylate / caprate) and the like.

The hydrocarbon oil includes hydrogenated polyisobutene, liquid paraffin, light liquid isoparaffin, squalane, phytosqualane, isododecane, alkane and the like.

The vegetable oil includes avocado oil, almond oil, olive oil, wheat germ oil, rice germ oil, rice bran oil, safflower oil, soybean oil, corn oil, rapeseed oil, palm oil, palm kernel oil, castor oil, sunflower oil, jojoba oil, macadamia nut oil, coconut oil and the like, and the animal oil includes EPA oil, DHA oil, beef tallow, chicken fat, lard, lanolin and the like.

The fat-soluble vitamin includes vitamin E such as tocopherol (tocopherol acetate), α-tocopherol, β-tocopherol, γ-tocopherol, δ-tocopherol, α-tocotrienol, β-tocotrienol, γ-tocotrienol, and δ-tocotrienol, vitamin A such as retinol and β-carotene and the like.

The ceramide includes ceramide NP, ceramide NG, ceramide AP, ceramide 2, and ceramide 3 and the like.

The preservative and antibacterial agent include glyceryl monocaprylate, glyceryl monocaprate, glyceryl monolaurate, methylparaben, propylparaben, ethylhexylglycerin, caprylyl glycol and the like.

The content of the poorly water-soluble substance in the cosmetic composition of the embodiment is preferably from 0.01 to 3% by mass and more preferably from 0.1 to 2% by mass. Incidentally, the content of the poorly water-soluble substances in the case where two or more kinds of poorly water-soluble substances are used refers to a total amount of the poorly water-soluble substances.

In the cosmetic composition of the embodiment, ingredients which are generally used in cosmetics other than the above ingredients can be appropriately formulated depending upon the use or purpose thereof. These optional ingredients include, for example, water, surfactants, polyhydric alcohol, aqueous gelation agents, oily gelation agents, ultraviolet absorbents, powders, antioxidants, preservatives, perfumes, colorants, chelating agents, refreshing agents, thickening agents, plant extracts, vitamins, neutralizing agents, moisturizing agents, antiinflammatory agents, pH adjusting agents, amino acids and the like.

The surfactant includes, but not particularly limited to, for example, polyoxyethylene fatty acid ester, polyoxyethylene glycerol fatty acid ester, polyoxyethylene sorbitan fatty acid ester, polyoxyethylene alkyl ether, polyoxyethylene (hydrogenated) castor oil, cocoyl glutamate Na, cocoyl glutamate TEA, lauroyl glutamate Na, potassium cocoyl glycine K, lauroyl methylalanine Na, methyl cocoyl taurate Na, lauroyl aspartate Na and the like.

The polyhydric alcohol includes, but not particularly limited to, for example, glycerol, diglycerol, ethylhexylglycerin, 1,3-butylene glycol (BG), propylene glycol (PG), propanediol, dipropylene glycol (DPG), pentylene glycol, 1,2-hexanediol, caprylyl glycol, 1,10-decanediol, isopentyldiol, sorbitol, mannitol, maltitol, xylitol and the like.

The cosmetic composition of the embodiment can be prepared by mixing the above ingredients according to a conventional method.

The cosmetic composition of the embodiment can be suitably used in various cosmetics. In other words, the present invention provides cosmetics containing the cosmetic composition of the embodiment. Here, the cosmetic composition of the embodiment may be directly used as cosmetics, or those further containing optional ingredients mentioned above can be made as cosmetics.

The cosmetics include, for example, skin toner, beauty serum, perfume, mouthwash, hair mist, cleansing skin toner, cleansing sheet, deodorant sheet and the like.

The method of producing the cosmetics includes a method of producing cosmetics including the step of containing each of the above ingredients. Here, "step of containing each of the above ingredients" includes an embodiment of adding the previously prepared cosmetic composition, and additionally an embodiment of separately formulating each of the above ingredients and preparing.

(Composition for foodstuff)

The composition for foodstuff of the embodiment contains the polyglycerol fatty acid ester of the present invention and the poorly water-soluble substance.

The content of the polyglycerol fatty acid ester of the present invention in the composition for foodstuff of the embodiment is preferably from 0.5 to 40% by mass, more preferably from 1 to 20% by mass, and even more preferably from 1 to 10% by mass.

The content of the poorly water-soluble substance in the composition for foodstuff of the embodiment is preferably from 1 to 20% by mass and more preferably from 1 to 10% by mass. Incidentally, the content of the poorly water-soluble substances in the case where two or more kinds of poorly water-soluble substances are used refers to a total amount of the poorly water-soluble substances.

In the composition for foodstuff of the embodiment, ingredients which are generally used in foodstuff other than the above ingredients can be appropriately formulated depending upon the use or purpose thereof. These optional ingredients include, for example, water, surfactants, polyhydric alcohol, powders, antioxidants, preservatives, perfumes, colorants, chelating agents, refreshing agents, thickening agents, plant extracts, vitamins, neutralizing agents, pH adjusting agents, amino acids and the like.

The composition for foodstuff of the embodiment can be prepared by mixing the above ingredients according to a conventional method.

The composition for foodstuff of the embodiment can be suitably used in various foodstuff. The peculiar flavor of surfactants can be reduced by transparently dissolving the poorly water-soluble substance with a small amount of surfactants. In other words, the present invention provides foodstuff with good flavor containing the composition for foodstuff of the embodiment.

The foodstuff includes, but not particularly limited to, for example, beverages such as fruit juice-free beverages, fruit juice-containing beverages, vegetable beverages, carbonated beverages, coffee, alcoholic beverages, mineral-containing beverages, vitamin-containing beverages, and beverages containing functional food materials, milk and products containing milk as a main raw material such as milk beverages, probiotic beverages, fermented milk, yogurt, ice cream, dessert food such as jelly, bavarois, and pudding, heat-treated processed food and pre-packed food such as beef bowl, pork bowl, chicken and eggs bowl, chuka-don (a bowl of rice with a chop-suey-like mixture on it), soup, pasta sauce, curry, stew, rice porridge, canned beverages, canned food, bottled beverages, and jarred food, confectioneries such as candies and snack food. Especially, the composition for foodstuff of the embodiment is suitable for foodstuff which transparency is needed such as beverages such as fruit juice-free beverages, fruit juice-containing beverages, vegetable beverages, carbonated beverages, coffee, alcoholic beverages, mineral-containing beverages, vitamin-containing beverages, and beverages containing functional food materials.

The method of producing the foodstuff includes a method of production including the step of containing each of the above ingredients. Here, "step of containing each of the above ingredients" includes an embodiment of adding the previously prepared composition for foodstuff, and additionally an embodiment of separately formulating each of the above ingredients and preparing.

## EXAMPLES

Hereinafter, the present invention will be particularly described by showing Examples and Comparative Examples, without intending to limit the scope of the present invention to the following Examples.

### Polyglycerol Production Example 1

To a three-necked flask equipped with Dimroth and a stirrer, 100 g of glycerol and an alkaline catalyst were added, and the temperature was elevated to 120°C. Glycerol triglycidyl ether in an amount of 28.26 g was added thereto dropwise at a rate of 10 g/hr, and after completion of addition dropwise, the mixture was reacted for additional 1 hour. Thereafter, the solution passed through an ion-exchange resin column, and unreacted glycerol was removed. The resulting fraction was concentrated to provide polyglycerol 1.

### Polyglycerol Production Example 2

To a three-necked flask equipped with Dimroth and a stirrer, 100 g of glycerol and an alkaline catalyst were added, and the temperature was elevated to 120°C. Glycerol triglycidyl ether in an amount of 47.1 g was added thereto dropwise at a rate of 10 g/hr, and after completion of addition dropwise, the mixture was reacted for additional 1 hour. Thereafter, the solution passed through an ion-exchange resin column, and unreacted glycerol was removed. The resulting fraction was concentrated to provide polyglycerol 2.

### Polyglycerol Production Example 3

To a three-necked flask equipped with Dimroth and a stirrer, 100 g of diglycerol and an alkaline catalyst were added, and the temperature was elevated to 140°C. Glycerol triglycidyl ether in an amount of 19.56 g was added thereto dropwise at a rate of 10 g/hr, and after completion of addition dropwise, the mixture was reacted for additional 1 hour. Thereafter, the solution passed through an ion-exchange resin column, and unreacted diglycerol was removed. The resulting fraction was concentrated to provide polyglycerol 3.

### Polyglycerol Production Example 4

To a three-necked flask equipped with Dimroth and a stirrer, 100 g of diglycerol and an alkaline catalyst were added, and the temperature was elevated to 140°C. Glycerol triglycidyl ether in an amount of 39.13 g was added thereto dropwise at a rate of 10 g/hr, and after completion of addition dropwise, the mixture was reacted for additional 1 hour. Thereafter, the solution passed through an ion-exchange resin column, and unreacted diglycerol was removed. The resulting fraction was concentrated to provide polyglycerol 4.

### Polyglycerol Production Example 5

To a three-necked flask equipped with Dimroth and a stirrer, 100 g of diglycerol and an alkaline catalyst were added, and the temperature was elevated to 140°C. Glycerol triglycidyl ether in an amount of 78.26 g was added thereto dropwise at a rate of 10 g/hr, and after completion of addition dropwise, the mixture was reacted for additional 1 hour. Thereafter, the solution passed through an ion-exchange resin column, and unreacted diglycerol was removed. The resulting fraction was concentrated to provide polyglycerol 5.

Polyglycerol Production Example 6

To a three-necked flask equipped with Dimroth and a stirrer, 100 g of glycerol and an alkaline catalyst were added, and the temperature was elevated to 140°C. Glycerol triglycidyl ether in an amount of 141.3 g was added thereto dropwise at a rate of 10 g/hr, and after completion of addition dropwise, the mixture was reacted for additional 1 hour. Thereafter, the solution passed through an ion-exchange resin column, and unreacted glycerol was removed. The resulting fraction was concentrated to provide polyglycerol 6.

Polyglycerols 7 and 8

Additionally, "polyglycerol #750," manufactured by Sakamoto Yakuhin Kogyo Co., LTD. was used as polyglycerol 7, and "polyglycerol #500," manufactured by Sakamoto Yakuhin Kogyo Co., LTD. was used as polyglycerol 8.

Polyglycerol Production Example 9

To a three-necked flask equipped with Dimroth and a stirrer, 100 g of glycerol and an alkaline catalyst were added, and the temperature was elevated to 120°C. Glycerol triglycidyl ether in an amount of 28.26 g was added thereto dropwise at a rate of 10 g/hr, and after completion of addition dropwise, the mixture was reacted for additional 1 hour. Thereafter, the solution passed through an ion-exchange resin column twice, and unreacted glycerol was removed as much as possible. The resulting fraction was concentrated to provide polyglycerol 9.

Polyglycerol Production Examples 10 to 13

A glycerol or diglycerol was added to the polyglycerol 9 to prepare polyglycerol 10 to 13. The contents of the glycerol or diglycerol are described in Table 1.

< Measurement of Ratio of Branched Glycerol and Number of Branched Glycerol per Molecule >

The ratio of branched glycerol and the number of branched glycerol per molecule were calculated as follows by 13C-NMR. The result is given in Table 1. In addition, 13C-NMR spectrum of polyglycerol 1 is shown in FIG. 1 as a representative example of 13C-NMR spectrum.

Pretreatment: 200 mg of polyglycerol was dissolved in 0.6 mL of heavy water
Apparatus used: 800 MHz 13C-NMR, "JNM-ECZ800R" manufactured by JEOL Ltd.
Cumulative number: 256 times
Measurement condition: temperature 30°C, measurement sequence single pulse 1H decoupling, pulse repetition time 7 seconds, and measurement is carried out using acetone as a standard peak (δ: 30.89 ppm)
Each peak range used for integral:
Total Integrals (TI): 60.0 - 83.0 ppm
Dendritic (D): 76.5 - 80.0 ppm

Method of Calculating Ratio of Branched Glycerol

TI shows integral values of all carbon atoms regarding polyglycerol, and D shows integral values of secondary carbon atoms regarding branched glycerol. The ratio of branched glycerol (%) is calculated from integral values of each peak mentioned above according to the following formula

$$\text{Ratio of branched glycerol (\%)} = (D \times 3 / TI) \times 100$$

Method of Calculating Number of Branched Glycerol per Molecule

The number of branched glycerol per molecule of polyglycerol is calculated according to the following formula

$$\text{Number of branched glycerol per molecule} = (D \times 3 / TI) \times \text{average degree of polymerization}$$

< Measurement of Content of Glycerol and Diglycerol >

The contents of glycerol and diglycerol in a polyglycerol was measured by making polyglycerol as a derivative according to trimethylsilylation, and carrying out separation and quantification of the polyglycerol derivative according to GC method (gas chromatograph method). The result is given in Table 1.

Apparatus used: GC-14B (SHIMADZU CORPORATION)

Column: OL-17 (50% phenyl methyl silicone, GL Sciences Inc.)

Column size: 0.5 m × 3 mm

Measurement temperature: 100 → 300°C

Heating rate: 10°C/min

[Table 1]

Table 1

| | Ratio of branched glycerol group (%) | Average degree of polymerization | Number of the branched glycerol group per molecule | Amount of glycerol | Amount of diglycerol |
|---|---|---|---|---|---|
| Polyglycerol 1 | 34.0 | 5.2 | 1.77 | 17.8 | 0.0 |
| Polyglycerol 2 | 31.1 | 6.2 | 1.93 | 10.3 | 0.0 |
| Polyglycerol 3 | 17.3 | 8.0 | 1.38 | 0.0 | 11.5 |
| Polyglycerol 4 | 26.2 | 12.3 | 3.22 | 0.0 | 4.8 |
| Polyglycerol 5 | 33.0 | 20.5 | 6.77 | 0.0 | 2.5 |
| Polyglycerol 6 | 55.0 | 24.3 | 13.37 | 3.2 | 0.0 |
| Polyglycerol 7 | 5.6 | 10.0 | 0.56 | 3.1 | 11.8 |
| Polyglycerol 8 | 4.7 | 6.0 | 0.28 | 0.1 | 6.7 |
| Polyglycerol 9 | 34.1 | 6.8 | 2.32 | 0.2 | 0.0 |
| Polyglycerol 10 | 32.1 | 6.3 | 2.02 | 4.9 | 0.0 |
| Polyglycerol 11 | 32.0 | 6.5 | 2.08 | 0.2 | 5.2 |
| Polyglycerol 12 | 25.6 | 5.4 | 1.38 | 25.2 | 0.0 |
| Polyglycerol 13 | 25.9 | 5.8 | 1.50 | 0.1 | 24.5 |

Preparation of Polyglycerol Fatty Acid Ester

Examples 1 to 18 and Comparative Examples 1 to 10

Polyglycerol fatty acid ester of each Example and Comparative Example was prepared as shown in Tables 2 to 4. A method of preparing Example 1 is more specifically explained as a representative example. To a four-necked flask equipped with a stirrer, 32.4 g of polyglycerol 1 obtained in Polyglycerol Production Example 1, 7.6 g of lauric acid and an alkaline catalyst were added, and esterification reaction was carried out at 250°C. The reaction was continued until the acidic value of a reactant achieved 0.5 or less, to provide a polyglycerol fatty acid ester of Example 1. Other Examples and Comparative Examples were also prepared according to the same procedure. In Examples 16 to 18 and Comparative Examples 5, 7 and 8, esterification reaction was carried out at 260°C. Incidentally, the HLB of the polyglycerol fatty acid ester can be adjusted by changing the ratio of polyglycerol and fatty acid. For example, reaction and preparation were carried out using 70 g of polyglycerol and 30 g of lauric acid in the case of Example 12 (HLB 13.8), 88.1 g of polyglycerol and

11.9 g of lauric acid in the case of Example 13 (HLB 17.6), and 93 g of polyglycerol and 7 g of lauric acid in the case of Example 14 (HLB 18.6).

< Test Example 1: Solubilization Test 1 >

Polyglycerol fatty acid esters of Examples 1 to 16 and Comparative Examples 1 to 8 in an amount of 0.5 g and an appropriate amount of lavender oil were mixed at 50°C and the mixture was stirred and poured into 100 g of water at 70°C. After stirring for 5 minutes, the mixture was cooled to 25°C to measure the transmittance at the wavelength of 650nm by the Spectrophotometer (manufactured by Shimadzu Corporation: UV-260). The amount of lavender oil in the case where the transmittance was 98% or more was considered as the solubilizing amount. The results are given in Tables 2 and 3.

[Table 2]

Table 2

| | Polyglycerol used | Fatty acid | HLB | Solubilizing amount(g) Poorly water-soluble substance: Lavender oil |
|---|---|---|---|---|
| Ex. 1 | Polyglycerol 1 | Lauric acid | 16.1 | 0.25 |
| Ex. 2 | Polyglycerol 2 | Lauric acid | 16.1 | 0.25 |
| Ex. 3 | Polyglycerol 3 | Lauric acid | 16.1 | 0.30 |
| Ex. 4 | Polyglycerol 4 | Lauric acid | 16.1 | 0.35 |
| Ex. 5 | Polyglycerol 5 | Lauric acid | 16.1 | 0.30 |
| Ex. 6 | Polyglycerol 6 | Lauric acid | 16.1 | 0.20 |
| Comp. Ex. 1 | Polyglycerol 7 | Lauric acid | 16.1 | 0.10 |
| Comp. Ex. 2 | Polyglycerol 8 | Lauric acid | 16.1 | 0.15 |
| Ex. 7 | Polyglycerol 9 | Lauric acid | 16.1 | 0.30 |
| Ex. 8 | Polyglycerol 10 | Lauric acid | 16.1 | 0.30 |
| Ex. 9 | Polyglycerol 11 | Lauric acid | 16.1 | 0.30 |
| Ex. 10 | Polyglycerol 12 | Lauric acid | 16.1 | 0.20 |
| Ex. 11 | Polyglycerol 13 | Lauric acid | 16.1 | 0.20 |

[Table 3]

Table 3

| | Polyglycerol used | Fatty acid | HLB | Solubilizing amount(g) Poorly water-soluble substance: Lavender oil |
|---|---|---|---|---|
| Ex. 12 | Polyglycerol 4 | Lauric acid | 13.8 | 0.35 |
| Ex. 13 | Polyglycerol 4 | Lauric acid | 17.6 | 0.30 |
| Ex. 14 | Polyglycerol 4 | Lauric acid | 18.6 | 0.25 |
| Ex. 15 | Polyglycerol 4 | Myristic acid | 15.7 | 0.35 |
| Ex. 16 | Polyglycerol 4 | Oleic acid | 14.9 | 0.20 |
| Comp. Ex. 3 | Polyglycerol 4 | Lauric acid | 12.0 | Insoluble |
| Comp. Ex. 4 | Polyglycerol 7 | Lauric acid | 13.8 | 0.10 |
| Comp. Ex. 5 | Polyglycerol 7 | Lauric acid | 17.6 | 0.10 |

(continued)

| | Polyglycerol used | Fatty acid | HLB | Solubilizing amount(g) Poorly water-soluble substance: Lavender oil |
|---|---|---|---|---|
| Comp. Ex. 6 | Polyglycerol 7 | Lauric acid | 18.6 | 0.10 |
| Comp. Ex. 7 | Polyglycerol 7 | Myristic acid | 15.7 | 0.15 |
| Comp. Ex. 8 | Polyglycerol 7 | Oleic acid | 14.9 | 0.10 |

< Test Example 2: Solubilization Test 2 >

Polyglycerol fatty acid esters of Examples 16 to 18 and Comparative Examples 8 to 10 in an amount of 0.5 g and an appropriate amount of cetyl ethylhexanoate were mixed at 50°C and the mixture was stirred and poured into 100 g of water at 70°C. After stirring for 5 minutes, the mixture was cooled to 25°C to measure the transmittance at the wavelength of 650nm by the Spectrophotometer (manufactured by Shimadzu Corporation: UV-260). The amount of cetyl ethylhexanoate in the case where the transmittance was 98% or more was considered as solubilizing amount. The results are given in Table 4.

< Test Example 3: Solubilization Test 3 >

Polyglycerol fatty acid esters of Examples 16 to 18 and Comparative Examples 8 to 10 in an amount of 0.5 g and an appropriate amount of phytosqualane were mixed at 50°C and the mixture was stirred and poured into 100 g of water at 70°C. After stirring for 5 minutes, the mixture was cooled to 25°C to measure the transmittance at the wavelength of 650nm by the Spectrophotometer (manufactured by Shimadzu Corporation: UV-260). The amount of phytosqualane in the case where the transmittance was 98% or more was considered as solubilizing amount. The results are given in Table 4.

[Table 4]

Table 4

| | Polyglycerol used | Fatty acid | HLB | Solubilizing amount(g) Poorly water-soluble substance: Cetyl ethylhexanoate | Solubilizing amount(g) Poorly water-soluble substance: Phytosqualane |
|---|---|---|---|---|---|
| Ex. 16 | Polyglycerol 4 | Oleic acid | 14.9 | 0.35 | 0.10 |
| Ex. 17 | Polyglycerol 4 | Stearic acid | 16.5 | 0.35 | 0.14 |
| Ex. 18 | Polyglycerol 4 | Isostearic acid | 14.9 | 0.30 | 0.12 |
| **Comp.** Ex. 8 | Polyglycerol 7 | Oleic acid | 14.9 | Insoluble | Insoluble |
| Comp. Ex. 9 | Polyglycerol 7 | Stearic acid | 16.5 | Insoluble | Insoluble |
| Comp. Ex. 10 | Polyglycerol 7 | Isostearic acid | 14.9 | Insoluble | Insoluble |

Details of components described in Tables 2 to 4 are shown below. Lavender oil: manufactured by Ogawa & Co., Ltd.

Cetyl ethylhexanoate: manufactured by Kao Corporation

Phytosqualane: manufactured by SOPHIM

As shown in Tables 2 to 4, all of Examples 1 to 18 using the polyglycerol fatty acid ester of the present invention had an excellent capability of solubilizing poorly water-soluble substances as compared to compositions of Comparative Examples 1, 2, and 4 to 10 prepared using commercially available polyglycerol 7 and 8, and composition of Comparative Example 3 having a low HLB.

Preparation of Skin Toner

A skin toner was prepared by the composition shown in Table 5 using the polyglycerol fatty acid ester of the present invention according to a conventional method. The transmittance of the obtained skin toner was measured in the same manner as Test Examples 1 to 3. The results are shown in Table 5. In addition, when the skin toner was applied to the skin, the usability was suitable without stickiness on drying.

[Table 5]

| Table 5: Skin toner | |
|---|---|
| Name of raw material | Formulated proportion % by weight |
| Ex. 4 | 0.20 |
| Lavender oil | 0.10 |
| 1,3BG | 5.00 |
| Glycerol | 5.00 |
| Caprylyl glycol | 0.40 |
| Citric acid | 0.01 |
| Trisodium citrate | 0.09 |
| Water | 89.20 |
| Transmittance (%T) 650nm | 99.12 |

The transparency of appearance was maintained, and the usability was suitable with lower stickiness on drying after use.

Preparation of Composition for Foodstuff 1

To 7.8 g of glycerol as a polyhydric alcohol, 1 g of ion exchanged water and 0.4 g of the polyglycerol fatty acid ester of Example 14 were added, and the mixture was heated to 65°C and dissolved. Extracted tocopherol in an amount of 0.8 g was added as a poorly water-soluble substance (antioxidant), the mixture was finely emulsified by a homo mixer under a pressure of 150 MPa, thereby obtaining the composition for foodstuff 1.

Preparation of Composition for Foodstuff 2

The composition for foodstuff 2 was obtained in the same manner as mentioned above except that 0.4 g of the polyglycerol fatty acid ester of Comparative Example 7 was added in place of the polyglycerol fatty acid ester of Example 14.

< Test Example 4: Solubilization Test 4 >

Composition for foodstuff in an amount of 0.1 g was solubilized in 100 g of water, then transmittance was measured at the wavelength of 650nm by the Spectrophotometer (manufactured by Shimadzu Corporation: UV-260). The results are shown in Table 6.

< Test Example 5: Measurement of Particle Size >

Composition for foodstuff in an amount of 0.1 g was solubilized in 100 g of water, then the average particle size was measured by the particle size analyzer (manufactured by Beckman Coulter, Inc.: L-230). The results are shown in Table 6.

[Table 6]

Table 6

| | Polyglycerol fatty acid ester used | Transmittance (%) | Average particle size (nm) |
|---|---|---|---|
| Composition for foodstuff 1 | Example 14 | 98.5 | 80 |
| Composition for foodstuff 2 | Comp. Ex. 7 | 85.1 | 350 |

As shown in Table 6, it is clear that the composition for foodstuff using the polyglycerol fatty acid ester of Example 14 has a high transmittance when solubilized in water, and has excellent transparency. In addition, since the average particle size of the poorly water-soluble substance is minute, it can be said that the composition has an excellent long-term stability.

INDUSTRIAL APPLICABILITY

According to the present invention, the polyglycerol fatty acid ester having an excellent capability of solubilizing poorly water-soluble substance can be provided, and the polyglycerol fatty acid ester can be suitably used in application such as cosmetics and foodstuff.

## Claims

1. A polyglycerol fatty acid ester which is an ester of a polyglycerol of which the ratio of a branched glycerol group represented by the following formula (1):

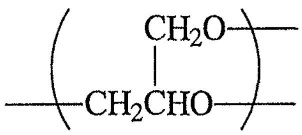

(1)

is from 14 to 60% and an average degree of polymerization is from 5 to 25, and a fatty acid having the number of carbon atoms of from 6 to 22, wherein an HLB is 13 or more.

2. The polyglycerol fatty acid ester according to claim 1, wherein the total content of a glycerol and a diglycerol in the polyglycerol is from 0.01 to 30% by mass.

3. The polyglycerol fatty acid ester according to claim 1, wherein the number of the branched glycerol group per polyglycerol molecule is from 1 to 15 on average.

4. The polyglycerol fatty acid ester according to claim 1, wherein the fatty acid is lauric acid, myristic acid, oleic acid, stearic acid, and isostearic acid.

5. A solubilizing agent comprising a polyglycerol fatty acid ester which is an ester of a polyglycerol of which the ratio of a branched glycerol group represented by the following formula (1):

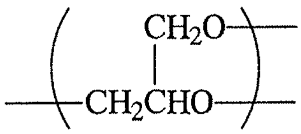

(1)

is from 14 to 60% and an average degree of polymerization is from 5 to 25, and a fatty acid having the number of carbon atoms of from 6 to 22, wherein an HLB is 13 or more.

6. A cosmetic composition comprising:

a polyglycerol fatty acid ester which is an ester of a polyglycerol of which the ratio of a branched glycerol group

represented by the following formula (1):

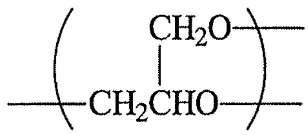

(1)

is from 14 to 60% and an average degree of polymerization is from 5 to 25, and a fatty acid having the number of carbon atoms of from 6 to 22, wherein an HLB is 13 or more; and
a poorly water-soluble substance.

**7.** Cosmetics comprising the polyglycerol fatty acid ester as defined in any one of claims 1 to 4 or the cosmetic composition as defined in claim 6.

**8.** A composition for foodstuff comprising:

a polyglycerol fatty acid ester which is an ester of a polyglycerol of which the ratio of a branched glycerol group represented by the following formula (1):

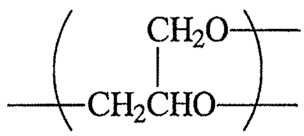

(1)

is from 14 to 60% and an average degree of polymerization is from 5 to 25, and a fatty acid having the number of carbon atoms of from 6 to 22, wherein an HLB is 13 or more; and
a poorly water-soluble substance.

**9.** Foodstuff comprising the polyglycerol fatty acid ester as defined in any one of claims 1 to 4 or the composition for foodstuff as defined in claim 8.

[FIG. 1]

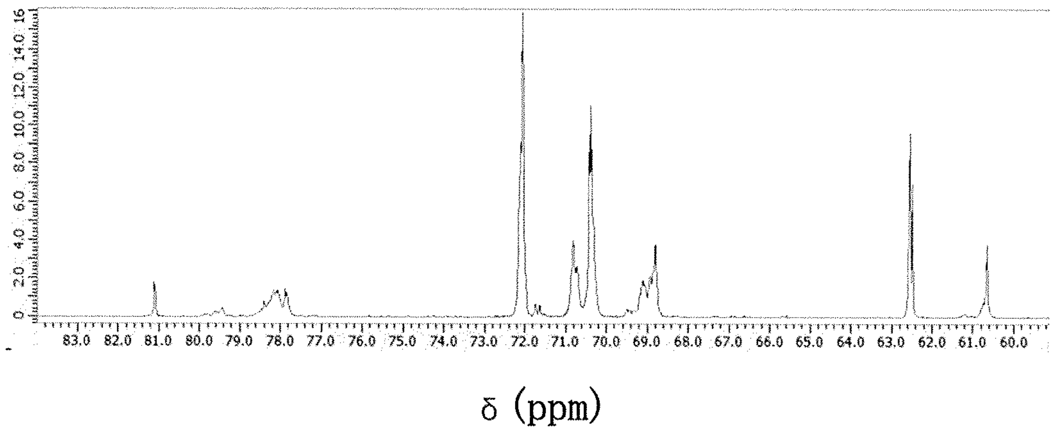
0
2.0
4.0
6.0
8.0
10.0
12.0
14.0
16
83.0 82.0 81.0 80.0 79.0 78.0 77.0 76.0 75.0 74.0 73.0 72.0 71.0 70.0 69.0 68.0 67.0 66.0 65.0 64.0 63.0 62.0 61.0 60.0
δ (ppm)

**INTERNATIONAL SEARCH REPORT**

International application No. **PCT/JP2023/038340**

**A. CLASSIFICATION OF SUBJECT MATTER**

***A61K 8/86***(2006.01)i; ***A23L 2/52***(2006.01)i; ***A23L 33/10***(2016.01)i; ***A23L 33/105***(2016.01)i; ***A23L 33/12***(2016.01)i; ***A61K 8/37***(2006.01)i; ***A61K 8/39***(2006.01)i; ***A61Q 19/00***(2006.01)i; ***A61Q 19/10***(2006.01)i; ***C07C 69/33***(2006.01)i; ***C08G 65/332***(2006.01)i; ***C08K 5/00***(2006.01)i; ***C08L 71/00***(2006.01)i

FI: A61K8/86; A61K8/39; A61Q19/10; A61K8/37; C08G65/332; A61Q19/00; A23L33/10; A23L33/12; A23L2/00 F; A23L2/52; A23L33/105; C08L71/00; C08K5/00; C07C69/33 CSP

According to International Patent Classification (IPC) or to both national classification and IPC

**B. FIELDS SEARCHED**

Minimum documentation searched (classification system followed by classification symbols)

A61K8/86; A23L2/52; A23L33/10; A23L33/105; A23L33/12; A61K8/37; A61K8/39; A61Q19/00; A61Q19/10; C07C69/33; C08G65/332; C08K5/00; C08L71/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2006-346526 A (TAIYO KAGAKU CO., LTD.) 28 December 2006 (2006-12-28) entire text | 1-9 |
| A | JP 2007-209251 A (TAIYO KAGAKU CO., LTD.) 23 August 2007 (2007-08-23) entire text | 1-9 |
| A | JP 2001-064236 A (TAIYO KAGAKU CO., LTD.) 13 March 2001 (2001-03-13) entire text | 1-9 |
| A | JP 2006-213698 A (RIKEN VITAMIN CO., LTD.) 17 August 2006 (2006-08-17) entire text | 1-9 |
| A | JP 9-238619 A (TAIYO KAGAKU CO., LTD.) 16 September 1997 (1997-09-16) entire text | 1-9 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/038340**

| Patent document cited in search report | Publication date (day/month/year) | Patent family member(s) | Publication date (day/month/year) |
|---|---|---|---|
| JP 2006-346526 A | 28 December 2006 | US 2009/0018358 A1<br>entire text<br>EP 1801096 A1 | |
| JP 2007-209251 A | 23 August 2007 | (Family: none) | |
| JP 2001-064236 A | 13 March 2001 | US 2002/0035238 A1<br>entire text | |
| JP 2006-213698 A | 17 August 2006 | EP 1679300 A1<br>entire text | |
| JP 9-238619 A | 16 September 1997 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018070529 A

### Non-patent literature cited in the description

- Standard Methods for the Analysis of Fats, Oils and Related Materials (I). Japan Oil Chemists' Society, 1996